# EUROPEAN PATENT APPLICATION

(11) **EP 4 606 810 A1**
(43) Date of publication of application: **27.08.2025**
(21) Application number: 24883599.3
(22) Date of filing: 22.05.2024
(51) Int. Cl.: C07H 3/02, C07H 1/06, B01D 15/36, C12P 19/02

(54) **METHOD FOR PURIFYING ALLULOSE-CONTAINING SOLUTION USING ION EXCHANGE**

(30) Priority: 19.12.2023 KR 20230185348
(71) Applicant: Daesang Corporation, Seoul 03130 (KR)
(72) Inventor: JUNG, Da Han, Seoul 07789 (KR); PARK, Tae Hyeon, Seoul 07789 (KR)
(74) Representative: Kehl, Ascherl, Liebhoff & Ettmayr Patentanwälte Partnerschaft mbB
(86) International application number: PCT/KR2024/006884
(87) International publication number: WO 2025/135326

(57) **Abstract**

An embodiment of the present disclosure provides a method for purifying an allulose-containing solution including obtaining an allulose-containing solution ion-purified by passing the allulose-containing solution through an ion exchange resin column filled with a mixed-bed ion exchange resin. In the method for purifying the allulose-containing solution according to an embodiment of the present disclosure, the mixed-bed ion exchange resin is a mixture of a strongly acidic cation exchange resin and a weakly basic anion exchange resin, and the weakly basic anion exchange resin constituting the mixed-bed ion exchange resin has an exchange capacity fraction of quaternary ammonium of 0 to 10%. According to the present disclosure, by using the method for purifying the allulose-containing solution, it is possible to not only lower the electrical conductivity of the allulose-containing solution to an acceptable level, but also suppress allulose from being converted to fructose or other substances due to functional groups of the ion exchange resin, thereby minimizing allulose loss. Therefore, the method for purifying the allulose-containing solution according to the present disclosure is suitable for mass-producing high-quality allulose.

## Description

### EXCHANGE

### [Technical Field]

The present disclosure relates to a method for purifying an allulose-containing solution, and more particularly, to a method for minimizing allulose loss when purifying an allulose-containing solution using an ion exchange resin.

### [Background Art]

D-allulose is also called D-psicose as an epimer of carbon at position 3 of fructose. The D-allulose is a functional monosaccharide that has 70% sweetness compared to sugar (Oshima 2006), but only 0.3% energy to be applied as a low-calorie sweetener in diet foods (Matsuo et al. 2002). In addition, the D-allulose has a function of suppressing glucose absorption and blood sugar and can be applied to food and drink for diabetic patients, food and drink for self-discipline, and the like, and may suppress the accumulation of abdominal fat through inhibition of enzyme activity involved in lipid synthesis in the liver and thus can be used for various functional foods such as health foods, and the like (Matsuo et al. 2001; Iida et al. 2008; Hayashi et al. 2010; Hossain et al. 2011).

Due to the characteristics, the allulose is a good source capable of replacing sugar, but belongs to a rare sugar, which is a monosaccharide that rarely exists in nature, and thus there is required a method of efficiently producing allulose to be applied to the food industry. The most efficient method for producing allulose for industrialization is a method for converting fructose to allulose using D-allulose 3-epimerase. A D-allulose-containing reaction product produced by an enzymatic reaction is then filtered, decolorized, concentrated, and purified through ion exchange to be commercialized in the form of a low-purity solution having a D-allulose content of about 20 to 30% (w/w) based on the solid content. In addition, the low-purity solution having the D-allulose content of about 20 to 30% (w/w) is commercialized in the form of a high-purity solution having a D-allulose content of about 95 to 99% (w/w) based on the solid content by simulated moving bed (SMB) chromatography separation and ion exchange purification.

With respect to the allulose purification technology, Korean Patent Publication No. 10-1988441 discloses a method for purifying allulose including mixing an allulose conversion reactant and powdered activated carbon, performing a solid-liquid separation process on the mixed solution to remove impurities and activated carbon, and obtaining a filtrate; performing an activated carbon treatment step, and then performing an ion purification process using a column filled with an ion exchange resin; and an allulose separation step of obtaining an allulose fraction and a fructose raffinate by performing a simulated moving bed (SMB) chromatography separation process.

### [Disclosure]

### [Technical Problem]

The present disclosure is derived under the conventional technical background, and an object of the present disclosure is to provide a method for purifying an allulose-containing solution capable of minimizing allulose loss while achieving an acceptable level of ion purification efficiency using an ion exchange resin.

### [Technical Solution]

To produce a low-purity solution-type product having a D-allulose content of about 20 to 30% (w/w) based on the solid content, when purifying a D-allulose-containing reaction product solution by ion exchange, some of allulose is converted to fructose or other substances due to functional groups of the ion exchange resin, resulting in allulose loss. In addition, in order to produce a high-purity solution-type product having a D-allulose content of about 95 to 99% (w/w) based on the solid content, when performing ion exchange purification after simulated moving bed (SMB) chromatography separation, a mixed-bed ion exchange resin consisting of a strongly acidic cation exchange resin and a strongly basic anion exchange resin is generally used, but in this case, a very large loss of allulose occurs. The present inventors recognized a unique problem occurring in a process of purifying an allulose-containing solution using ion exchange, and conducted an experiment of purifying an allulose-containing solution using various combinations of ion exchange resin columns to solve this problem, and as a result, confirmed that the loss of allulose was minimized when a specific combination of ion exchange resins was used, and then completed the present disclosure.

In order to solve the above problem, an embodiment of the present disclosure provides a method for purifying an allulose-containing solution including obtaining an allulose-containing solution ion-purified by passing the allulose-containing solution through an ion exchange resin column filled with a mixed-bed ion exchange resin. In the method for purifying the allulose-containing solution according to an embodiment of the present disclosure, the mixed-bed ion exchange resin is a mixture of a strongly acidic cation exchange resin and a weakly basic anion exchange resin, and the weakly basic anion exchange resin constituting the mixed-bed ion exchange resin has an exchange capacity fraction of quaternary ammonium of 0 to 10%.

In order to solve the above problem, another embodiment of the present disclosure provides a method for purifying an allulose-containing solution, including obtaining an allulose-containing solution ion-purified by sequentially passing the allulose-containing solution through a first ion exchange resin column filled with a strongly acidic cation exchange resin, a second ion exchange resin column filled with a weakly basic anion exchange resin, and a third ion exchange resin column filled with a mixed-bed ion exchange resin. In the method for purifying the allulose-containing solution according to another embodiment of the present disclosure, the mixed-bed ion exchange resin is a mixture of a strongly acidic cation exchange resin and a weakly basic anion exchange resin. In addition, in the method for purifying the allulose-containing solution according to another embodiment of the present disclosure, the weakly basic anion exchange resin filled in the second ion exchange resin column and the weakly basic anion exchange resin constituting the mixed-bed ion exchange resin have exchange capacity fractions of quaternary ammonium of 0 to 10%.

### [Advantageous Effects]

According to the present disclosure, by using the method for purifying the allulose-containing solution, it is possible to not only lower the electrical conductivity of the allulose-containing solution to an acceptable level, but also suppress allulose from being converted to fructose or other substances due to the functional groups of the ion exchange resin, thereby minimizing allulose loss. Therefore, the method for purifying the allulose-containing solution according to the present disclosure is suitable for mass-producing high-quality allulose.

### [Best Mode]

Hereinafter, the present disclosure will be described in detail.

The term 'exchange capacity fraction' used herein is an exchange capacity expressed as a percentage in which a specific exchange group contributes in the total exchange capacity of an ion exchange resin.

The present disclosure relates to a method for purifying an allulose-containing solution capable of minimizing allulose loss while achieving an acceptable level of ion purification efficiency using an ion exchange resin.

The method for purifying the allulose-containing solution according to an embodiment of the present disclosure includes obtaining an allulose-containing solution ion-purified by passing the allulose-containing solution through an ion exchange resin column filled with a mixed-bed ion exchange resin. The mixed-bed ion exchange resin is a mixture of a strongly acidic cation exchange resin and a weakly basic anion exchange resin.

The types of strongly acidic cation exchange resin constituting the mixed-bed ion exchange resin are not significantly limited, and may be selected from, for example, hydrogen ion type (H-type) or sodium type (Na-type) strongly acidic cation exchange resins. The Na-type strongly acidic cation exchange resin may also be used after being converted to the H-type using an appropriate concentration of acid (e.g., HCl) aqueous solution before use. In addition, the strongly acidic cation exchange resin may be a gel type or a porous type, preferably a porous type. In addition, the strongly acidic cation exchange resin has a sulfonic acid group as an exchange group, and the matrix may be a styrene-divinylbenzene copolymer prepared by polymerizing monovinylidene aromatic monomers (e.g., styrene, etc.) and a crosslinking agent (e.g., divinylbenzene (DVB), etc.).

The type of weakly basic anion exchange resin constituting the mixed-bed ion exchange resin is not significantly limited as long as the exchange capacity fraction of quaternary ammonium is 0 to 10%. In addition, the weakly basic anion exchange resin may be a gel type or a porous type, preferably a porous type. In addition, the weakly basic anion exchange resin has a tertiary amine group or a quaternary ammonium group as an exchange group, and the matrix may be a styrene-divinylbenzene copolymer prepared by polymerizing monovinylidene aromatic monomers (e.g., styrene, etc.) and a crosslinking agent (e.g., divinylbenzene (DVB), etc.). The quaternary ammonium group, which is an exchange group present in the weakly basic anion exchange resin, is selected from a type I quaternary ammonium group such as trimethyl ammonium or a type II quaternary ammonium group such as dimethylethanol ammonium, and preferably a type I quaternary ammonium group such as trimethyl ammonium when considering purification efficiency and minimization of allulose loss. The weakly basic anion exchange resin constituting the mixed-bed ion exchange resin has a tertiary amine group as an exchange group when the exchange capacity fraction of quaternary ammonium is 0%, and the exchange capacity fraction of the tertiary amine group is 100%. In addition, the weakly basic anion exchange resin constituting the mixed-bed ion exchange resin has a quaternary ammonium group and a tertiary amine group as an exchange group when the exchange capacity fraction of quaternary ammonium is 5%, and the exchange capacity fraction of the tertiary amine group is 95%. In addition, the weakly basic anion exchange resin constituting the mixed-bed ion exchange resin has a quaternary ammonium group and a tertiary amine group as an exchange group when the exchange capacity fraction of quaternary ammonium is 10%, and the exchange capacity fraction of the tertiary amine group is 90%. Considering the ion purification efficiency, the weakly basic anion exchange resin constituting the mixed-bed ion exchange resin preferably has an exchange capacity fraction of quaternary ammonium of 5 to 10%.

In the method for purifying the allulose-containing solution according to an embodiment of the present disclosure, a mixed volume ratio of the strongly acidic cation exchange resin and the weakly basic anion exchange resin constituting the mixed-bed ion exchange resin is not significantly limited, and may be preferably 1 : 1 to 1 : 4, and more preferably 1 : 1.5 to 1 : 3 when considering the ion purification efficiency and minimization of allulose loss.

In the method for purifying the allulose-containing solution according to an embodiment of the present disclosure, the sugar solid concentration, pH, electrical conductivity, allulose content, etc. of the allulose-containing solution injected into the ion exchange resin column are not significantly limited. The allulose-containing solution preferably has the sugar solid concentration of 5 to 50 Brix, the pH of 3.5 to 5.5, the electrical conductivity of 5 to 80 *µs*/cm, and the allulose content of 90 to 99% (w/w) based on the total weight of the solid content, and more preferably the sugar solid concentration of 8 to 30 Brix, the pH of 4 to 5, the electrical conductivity of 15 to 60 *µs*/cm, and the allulose content of 95 to 99% (w/w) based on the total weight of the solid content, when considering the ion purification efficiency.

In the method for purifying the allulose-containing solution according to an embodiment of the present disclosure, the temperature of the ion exchange resin column is preferably 30 to 55°C, and more preferably 40 to 50°C, when considering the ion purification efficiency and minimization of allulose loss. In addition, in the method for purifying the allulose-containing solution according to an embodiment of the present disclosure, a space velocity (SV) when the allulose-containing solution passes through the ion exchange resin column is not significantly limited, and preferably 1 to 5, and more preferably 1.5 to 3 when considering the ion purification efficiency.

A method for purifying an allulose-containing solution according to another embodiment of the present disclosure includes obtaining an allulose-containing solution ion-purified by sequentially passing the allulose-containing solution through a first ion exchange resin column filled with a strongly acidic cation exchange resin, a second ion exchange resin column filled with a weakly basic anion exchange resin, and a third ion exchange resin column filled with a mixed-bed ion exchange resin.

In the method for purifying the allulose-containing solution according to another embodiment of the present disclosure, the types of strongly acidic cation exchange resin filled in the first ion exchange resin column are not significantly limited, and may be selected from, for example, hydrogen ion type (H-type) or sodium type (Na-type) strongly acidic cation exchange resins. The Na-type strongly acidic cation exchange resin may also be used after being converted to the H-type using an appropriate concentration of acid (e.g., HCl) aqueous solution before use. In addition, the strongly acidic cation exchange resin may be a gel type or a porous type, preferably a gel type. In addition, the strongly acidic cation exchange resin has a sulfonic acid group as an exchange group, and the matrix may be a styrene-divinylbenzene copolymer prepared by polymerizing monovinylidene aromatic monomers (e.g., styrene, etc.) and a crosslinking agent (e.g., divinylbenzene (DVB), etc.).

In the method for purifying the allulose-containing solution according to another embodiment of the present disclosure, the types of weakly basic anion exchange resin filled in the second ion exchange resin column are not significantly limited as long as the exchange capacity fraction of quaternary ammonium is 0 to 10%. For example, the weakly basic anion exchange resin may be a gel type or a porous type, preferably a porous type. In addition, the weakly basic anion exchange resin has a tertiary amine group or a quaternary ammonium group as an exchange group, and the matrix may be a styrene-divinylbenzene copolymer prepared by polymerizing monovinylidene aromatic monomers (e.g., styrene, etc.) and a crosslinking agent (e.g., divinylbenzene (DVB), etc.). The quaternary ammonium group, which is an exchange group present in the weakly basic anion exchange resin, is selected from a type I quaternary ammonium group such as trimethyl ammonium or a type II quaternary ammonium group such as dimethylethanol ammonium, and preferably a type I quaternary ammonium group such as trimethyl ammonium when considering purification efficiency and minimization of allulose loss. The weakly basic anion exchange resin has a tertiary amine group as an exchange group when the exchange capacity fraction of quaternary ammonium is 0%, and the exchange capacity fraction of the tertiary amine group is 100%. In addition, the weakly basic anion exchange resin has a quaternary ammonium group and a tertiary amine group as an exchange group when the exchange capacity fraction of quaternary ammonium is 5%, and the exchange capacity fraction of the tertiary amine group is 95%. In addition, the weakly basic anion exchange resin has a quaternary ammonium group and a tertiary amine group as an exchange group when the exchange capacity fraction of quaternary ammonium is 10%, and the exchange capacity fraction of the tertiary amine group is 90%. The weakly basic anion exchange resin filled in the second ion exchange resin column preferably has a tertiary amine group as an exchange group and the exchange capacity fraction of quaternary ammonium is 0% when considering ion purification efficiency and minimization of allulose loss.

In the method for purifying the allulose-containing solution according to another embodiment of the present disclosure, the mixed-bed ion exchange resin filled in the third ion exchange resin column is a mixture of a strongly acidic cation exchange resin and a weakly basic anion exchange resin. The weakly basic anion exchange resin constituting the mixed-bed ion exchange resin has an exchange capacity fraction of quaternary ammonium of 0 to 10% and preferably the exchange capacity fraction of quaternary ammonium of 5 to 10% when considering ion purification efficiency and minimization of allulose loss. In addition, a mixed volume ratio of the strongly acidic cation exchange resin and the weakly basic anion exchange resin constituting the mixed-bed ion exchange resin is preferably 1 : 1 to 1 : 4. In the method for purifying the allulose-containing solution according to another embodiment of the present disclosure, the technical characteristics of the mixed-bed ion exchange resin filled in the third ion exchange resin column are the same as those described in the method for purifying the allulose-containing solution according to one embodiment of the present disclosure, and therefore, a detailed description thereof will be omitted.

In the method for purifying the allulose-containing solution according to another embodiment of the present disclosure, the volume ratio of the strongly acidic cation exchange resin filled in the first ion exchange resin column, the weakly basic anion exchange resin filled in the second ion exchange resin column, and the mixed-bed ion exchange resin filled in the third ion exchange resin column is not significantly limited, and preferably 1 : (0.5 to 2) : (0.5 to 2), and more preferably 1 : (0.65 to 1.5) : (0.65 to 1.5) when considering the ion purification efficiency.

In the method for purifying the allulose-containing solution according to another embodiment of the present disclosure, the sugar solid concentration, pH, electrical conductivity, allulose content, etc. of the allulose-containing solution injected into the first ion exchange resin column are not significantly limited. The allulose-containing solution preferably has the sugar solid concentration of 25 to 65 Brix, the pH of 3.5 to 5.5, the electrical conductivity of 20 to 400 *µs*/cm, and the allulose content of 10 to 50% (w/w) based on the total weight of the solid content, and more preferably the sugar solid concentration of 40 to 60 Brix, the pH of 4 to 5, the electrical conductivity of 50 to 250 *µs*/cm, and the allulose content of 15 to 35% (w/w) based on the total weight of the solid content, when considering the ion purification efficiency.

In the method for purifying the allulose-containing solution according to another embodiment of the present disclosure, the temperatures of the first ion exchange resin column, the second ion exchange resin column, and the third ion exchange resin column are preferably 30 to 55°C, and more preferably 35 to 45°C, when considering the ion purification efficiency and minimization of allulose loss. In addition, in the method for purifying the allulose-containing solution according to another embodiment of the present disclosure, a space velocity (SV) when the allulose-containing solution passes through the first ion exchange resin column, the second ion exchange resin column, and the third ion exchange resin column is not significantly limited, and preferably 1 to 5, and more preferably 1.5 to 3 when considering the ion purification efficiency.

Hereinafter, the present disclosure will be described in more detail with reference to Examples. However, the following Examples are only for clearly illustrating the technical features of the present disclosure, but do not limit the protection scope of the present disclosure.

### 1. Information on ion exchange resins

Table 1 below summarized information on ion exchange resins used in Examples of the present disclosure. All ion exchange resin products listed in Table 1 below were commercially purchased.

**[Table 1]**

| Internal management name of ion exchange resin | DS1 | DS2 | DS3 | DS4 | DS5 | DS6 |
|---|---|---|---|---|---|---|
| Resin type | Strongly acidic cation exchange resin | Strongly acidic cation exchange resin | Medium basic anion exchange resin | Weakly basic anion exchange resin | Weakly basic anion exchange resin | Strongly basic anion exchange resin |
| Resin particle type | Gel type | Porous type | Porous type | Porous type | Porous type | Porous type |
| Resin matrix material | Styrene-divinylbenzene copolymer | Styrene-divinylbenzene copolymer | Styrene-divinylbenzene copolymer | Styrene-divinylbenzene copolymer | Styrene-divinylbenzene copolymer | Styrene-divinylbenzene copolymer |
| Resin particle uniformity | Uniform (monodisperse) | Uniform (monodisperse) | Uniform (monodisperse) | Uniform (monodisperse) | Non-uniform | Uniform (monodisperse) |
| Total exchange capacity (eq/L) | 2.1-2.2 | 1.6-1.8 | 1.3-1.4 | 1.6 | 1.7-2.0 | 1.0-1.1 |
| Functional group | Sulfonic acid | Sulfonic acid | Tertiary amine, Quaternary ammonium | Tertiary amine, Quaternary ammonium | Tertiary amine | Quaternary ammonium (Type II) |
| Exchange capacity fraction of quaternary ammonium | | | 25% | 5-10% | 0% | 100% |

### 2. Ion exchange purification of allulose-containing solution

### Experimental Example 1.

A fructose solution was contacted with immobilized allulose epimerase to perform an isomerization reaction. Thereafter, the reaction product solution was added with activated carbon, subjected to a decolorization reaction at 50°C, and then filtered and concentrated to prepare a low-purity allulose-containing solution having a sugar solid concentration of about 50 Brix, a pH of about 4.5, and an electrical conductivity of about 108 *µs*/*cm.* The allulose content in the low-purity allulose-containing solution was about 20.44% (w/w) based on the total weight of the solid content. A mixed-bed ion exchange resin was prepared by mixing a strongly acidic cation exchange resin (product name: DS2) and a weakly basic anion exchange resin (product name: DS4) in a volume ratio of 1 : 2. Thereafter, a first column filled with a strongly acidic cation exchange resin (product name: DS1), a second column filled with a weakly basic anion exchange resin (product name: DS5), and a third column filled with a mixed-bed ion exchange resin were sequentially connected to prepare an ion exchange resin column having a total of three stages. The volume ratio of the cation exchange resin filled in the first column, the anion exchange resin filled in the second column, and the mixed-bed ion exchange resin filled in the third column was 1 : 1.5 : 1. Thereafter, the temperature of the ion exchange resin column was maintained at 45°C, and the low-purity allulose-containing solution passed through the ion exchange resin column at a space velocity (SV) of 2.6 to obtain an ion-purified allulose-containing solution.

### Experimental Example 2.

A fructose solution was contacted with immobilized allulose epimerase to perform an isomerization reaction. Thereafter, the reaction product solution was added with activated carbon, subjected to a decolorization reaction at 50°C, and then filtered and concentrated to prepare a low-purity allulose-containing solution having a sugar solid concentration of about 50 Brix, a pH of about 4.5, and an electrical conductivity of about 118 *µs*/*cm.* The allulose content in the low-purity allulose-containing solution was about 20.30% (w/w) based on the total weight of the solid content. A mixed-bed ion exchange resin was prepared by mixing a strongly acidic cation exchange resin (product name: DS2) and a weakly basic anion exchange resin (product name: DS4) in a volume ratio of 1 : 2. Thereafter, a first column filled with a strongly acidic cation exchange resin (product name: DS1), a second column filled with a weakly basic anion exchange resin (product name: DS4), and a third column filled with a mixed-bed ion exchange resin were sequentially connected to prepare an ion exchange resin column having a total of three stages. The volume ratio of the cation exchange resin filled in the first column, the anion exchange resin filled in the second column, and the mixed-bed ion exchange resin filled in the third column was 1 : 1.5 : 1. Thereafter, the temperature of the ion exchange resin column was maintained at 45°C, and the low-purity allulose-containing solution passed through the ion exchange resin column at a space velocity (SV) of 2.6 to obtain an ion-purified allulose-containing solution.

### Experimental Example 3.

A fructose solution was contacted with immobilized allulose epimerase to perform an isomerization reaction. Thereafter, the reaction product solution was added with activated carbon, subjected to a decolorization reaction at 50°C, and then filtered and concentrated to prepare a low-purity allulose-containing solution having a sugar solid concentration of about 50 Brix, a pH of about 4.4, and an electrical conductivity of about 101 *µs*/cm*.* The allulose content in the low-purity allulose-containing solution was about 20.41 % (w/w) based on the total weight of the solid content. A mixed-bed ion exchange resin was prepared by mixing a strongly acidic cation exchange resin (product name: DS2) and a weakly basic anion exchange resin (product name: DS4) in a volume ratio of 1 : 2. Thereafter, a first column filled with a strongly acidic cation exchange resin (product name: DS1), a second column filled with a medium basic anion exchange resin (product name: DS3), and a third column filled with a mixed-bed ion exchange resin were sequentially connected to prepare an ion exchange resin column having a total of three stages. The volume ratio of the cation exchange resin filled in the first column, the anion exchange resin filled in the second column, and the mixed-bed ion exchange resin filled in the third column was 1 : 1.5 : 1. Thereafter, the temperature of the ion exchange resin column was maintained at 45°C, and the low-purity allulose-containing solution passed through the ion exchange resin column at a space velocity (SV) of 2.6 to obtain an ion-purified allulose-containing solution.

### Experimental Example 4.

A fructose solution was contacted with immobilized allulose epimerase to perform an isomerization reaction. Thereafter, the reaction product solution was added with activated carbon, subjected to a decolorization reaction at 50°C, and then filtered and concentrated to prepare a low-purity allulose-containing solution having a sugar solid concentration of about 50 Brix, a pH of about 4.5, and an electrical conductivity of about 105 *µs*/cm. The allulose content in the low-purity allulose-containing solution was about 20.39% (w/w) based on the total weight of the solid content. A mixed-bed ion exchange resin was prepared by mixing a strongly acidic cation exchange resin (product name: DS2) and a strongly basic anion exchange resin (product name: DS6) in a volume ratio of 1 : 2. Thereafter, a first column filled with a strongly acidic cation exchange resin (product name: DS1), a second column filled with a medium basic anion exchange resin (product name: DS3), and a third column filled with a mixed-bed ion exchange resin were sequentially connected to prepare an ion exchange resin column having a total of three stages. The volume ratio of the cation exchange resin filled in the first column, the anion exchange resin filled in the second column, and the mixed-bed ion exchange resin filled in the third column was 1 : 1.5 : 1. Thereafter, the temperature of the ion exchange resin column was maintained at 45°C, and the low-purity allulose-containing solution passed through the ion exchange resin column at a space velocity (SV) of 2.6 to obtain an ion-purified allulose-containing solution.

### Experimental Example 5.

A fructose solution was contacted with immobilized allulose epimerase to perform an isomerization reaction. Thereafter, the reaction product solution was added with activated carbon, subjected to a decolorization reaction at 50°C, then filtered and concentrated, and purified using ion exchange resins to prepare a low-purity allulose-containing solution. Thereafter, a simulated moving bed (SMB) chromatography separation process was performed on the low-purity allulose-containing solution using a column filled with a cation exchange resin attached with a calcium activator to prepare a high-purity allulose-containing solution having a sugar solid concentration of about 10 Brix, a pH of about 4.5, and an electrical conductivity of about 36 *µs*/cm*.* The allulose content in the high-purity allulose-containing solution was about 97.96% (w/w) based on the total weight of the solid content. A mixed-bed ion exchange resin was prepared by mixing a strongly acidic cation exchange resin (product name: DS2) and a weakly basic anion exchange resin (product name: DS4) in a volume ratio of 1 : 2. Thereafter, an ion exchange resin column filled with the mixed-bed ion exchange resin was prepared. Thereafter, the temperature of the ion exchange resin column was maintained at 45°C, and the high-purity allulose-containing solution passed through the ion exchange resin column at a space velocity (SV) of 2.6 to obtain an ion-purified allulose-containing solution.

### Experimental Example 6.

A fructose solution was contacted with immobilized allulose epimerase to perform an isomerization reaction. Thereafter, the reaction product solution was added with activated carbon, subjected to a decolorization reaction at 50°C, then filtered and concentrated, and purified using ion exchange resins to prepare a low-purity allulose-containing solution. Thereafter, a simulated moving bed (SMB) chromatography separation process was performed on the low-purity allulose-containing solution using a column filled with a cation exchange resin attached with a calcium activator to prepare a high-purity allulose-containing solution having a sugar solid concentration of about 10 Brix, a pH of about 4.5, and an electrical conductivity of about 40 *µs*/cm*.* The allulose content in the high-purity allulose-containing solution was about 98.3% (w/w) based on the total weight of the solid content. A mixed-bed ion exchange resin was prepared by mixing a strongly acidic cation exchange resin (product name: DS2) and a medium basic anion exchange resin (product name: DS3) in a volume ratio of 1 : 2. Thereafter, an ion exchange resin column filled with the mixed-bed ion exchange resin was prepared. Thereafter, the temperature of the ion exchange resin column was maintained at 45°C, and the high-purity allulose-containing solution passed through the ion exchange resin column at a space velocity (SV) of 2.6 to obtain an ion-purified allulose-containing solution.

### Experimental Example 7.

A fructose solution was contacted with immobilized allulose epimerase to perform an isomerization reaction. Thereafter, the reaction product solution was added with activated carbon, subjected to a decolorization reaction at 50°C, then filtered and concentrated, and purified using ion exchange resins to prepare a low-purity allulose-containing solution. Thereafter, a simulated moving bed (SMB) chromatography separation process was performed on the low-purity allulose-containing solution using a column filled with a cation exchange resin attached with a calcium activator to prepare a high-purity allulose-containing solution having a sugar solid concentration of about 10 Brix, a pH of about 4.5, and an electrical conductivity of about 36 *µs*/*cm.* The allulose content in the high-purity allulose-containing solution was about 97.63% (w/w) based on the total weight of the solid content. A mixed-bed ion exchange resin was prepared by mixing a strongly acidic cation exchange resin (product name: DS2) and a strongly basic anion exchange resin (product name: DS6) in a volume ratio of 1 : 2. Thereafter, an ion exchange resin column filled with the mixed-bed ion exchange resin was prepared. Thereafter, the temperature of the ion exchange resin column was maintained at 45°C, and the high-purity allulose-containing solution passed through the ion exchange resin column at a space velocity (SV) of 2.6 to obtain an ion-purified allulose-containing solution.

The combinations of the ion exchange resins used in Experimental Examples 1 to 7 were summarized in Table 2 below.

**[Table 2]**

| Classification of Experimental Example | Ion exchange resin combination (order of passage of allulose-containing solution) | | |
|---|---|---|---|
| | Cation exchange resin (first stage) | Anion exchange resin (second stage) | Mixed-bed ion exchange resin (third stage) |
| Experimental Example 1 | Strongly acidic cation exchange resin DS 1 | Weakly basic anion exchange resin DS5 | Strongly acidic cation exchange resin DS2 + Weakly basic anion exchange resin DS4 |
| Experimental Example 2 | Strongly acidic cation exchange resin DS 1 | Weakly basic anion exchange resin DS4 | Strongly acidic cation exchange resin DS2 + Weakly basic anion exchange resin DS4 |
| Experimental Example 3 | Strongly acidic cation exchange resin DS 1 | Medium basic anion exchange resin DS3 | Strongly acidic cation exchange resin DS2 + Weakly basic anion exchange resin DS4 |
| Experimental Example 4 | Strongly acidic cation exchange resin DS 1 | Medium basic anion exchange resin DS3 | Strongly acidic cation exchange resin DS2 + Strongly basic anion exchange resin DS6 |
| Experimental Example 5 | - | - | Strongly acidic cation exchange resin DS2 + Weakly basic anion exchange resin DS4 |
| Experimental Example 6 | - | - | Strongly acidic cation exchange resin DS2 + Medium basic anion exchange resin DS3 |
| Experimental Example 7 | - | - | Strongly acidic cation exchange resin DS2 + Strongly basic anion exchange resin DS6 |

The changes in physical properties of the allulose-containing solution before being injected into the ion exchange resin column and after being discharged through the ion exchange resin column were summarized in Table 3 below.

**[Table 3]**

| Classification of Experimental Example | Allulose-containing solution before injection | | | Allulose-containing solution after discharge | | | Allulose loss rate (%) |
|---|---|---|---|---|---|---|---|
| | pH | Conductivity (*µ*m/cm) | Allulose content (%) | pH | Conductivity (*µ*m/cm) | Allulose content (%) | |
| Experimental Example 1 | 4.5 | 108 | 20.44 | 6.72 | 1.25 | 20.35 | 0.44 |
| Experimental Example 2 | 4.5 | 118 | 20.30 | 6.51 | 1.23 | 19.91 | 1.92 |
| Experimental Example 3 | 4.4 | 101 | 20.41 | 6.42 | 0.86 | 19.82 | 2.89 |
| Experimental Example 4 | 4.5 | 105 | 20.39 | 6.72 | 1.01 | 17.57 | 13.83 |
| Experimental Example 5 | 4.5 | 36 | 97.96 | 6.4 | 1.2 | 97.54 | 0.43 |
| Experimental Example 6 | 4.5 | 40 | 98.30 | 5.4 | 1.02 | 96.00 | 2.34 |
| Experimental Example 7 | 4.5 | 36 | 97.63 | 4.43 | 0.8 | 83.83 | 14.14 |

| | | | | | | | |
|---|---|---|---|---|---|---|---|
| * Allulose content: Weight percentage (%, w/w) based on the total weight of solid content * Allulose loss rate: Rate at which allulose was converted into fructose or other substances by isomerization, etc. when passing through the ion exchange resin | | | | | | | |

As shown in Tables 2 and 3 above, the allulose-containing solution discharged through the ion exchange resin column showed acceptable levels of pH and conductivity regardless of the combinations of ion exchange resins. However, the allulose loss caused by isomerization, etc. when the allulose-containing solution was ion-purified through the ion exchange resin column showed a large difference depending on each combination of ion exchange resins. Specifically, the allulose loss was minimized in Experimental Examples 1, 2, and 5. The ion exchange resin columns used in Experimental Examples 1, 2, and 5 commonly included the mixed-bed ion exchange resin prepared by mixing the strongly acidic cation exchange resin (product name: DS2) and the weakly basic anion exchange resin (product name: DS4) in a volume ratio of 1 : 2. Meanwhile, the ion exchange resin column used in Experimental Example 3 also included the mixed-bed ion exchange resin prepared by mixing the strongly acidic cation exchange resin (product name: DS2) and the weakly basic anion exchange resin (product name: DS4) in a volume ratio of 1 : 2, but the allulose loss in Experimental Example 3 was somewhat large. This result was caused by a difference in the anion exchange resin located in the second stage of the ion exchange resin column. In Experimental Examples 1 and 2, the anion exchange resins located in the second stage were a weakly basic anion exchange resin (product name: DS5) in which the exchange capacity fraction of quaternary ammonium was 0% and a weakly basic anion exchange resin (product name: DS4) in which the exchange capacity fraction of quaternary ammonium was 5 to 10%, respectively, whereas in Experimental Example 3, the anion exchange resin located in the second stage was a medium basic anion exchange resin (product name: DS3) in which the exchange capacity fraction of quaternary ammonium was 25%. The results showed that the allulose loss rate also increased as the exchange capacity fraction of quaternary ammonium of the anion exchange resin located in the second stage of the ion exchange resin column increased, and in terms of mass production, it is preferable that the exchange capacity fraction of quaternary ammonium of the anion exchange resin located in the second stage of the ion exchange resin column was 0 to 10%.

As described above, the present disclosure has been described through Examples above, but the present disclosure is not necessarily limited thereto, and various modifications can be made without departing from the scope and spirit of the present disclosure. Therefore, the scope of the present disclosure should be construed to include all embodiments falling within the scope of claims appended hereto.

## Claims

1. A method for purifying an allulose-containing solution comprising obtaining an allulose-containing solution ion-purified by passing the allulose-containing solution through an ion exchange resin column filled with a mixed-bed ion exchange resin, wherein
the mixed-bed ion exchange resin is a mixture of a strongly acidic cation exchange resin and a weakly basic anion exchange resin, and
the weakly basic anion exchange resin constituting the mixed-bed ion exchange resin has an exchange capacity fraction of quaternary ammonium of 0 to 10%.

2. The method for purifying the allulose-containing solution of claim 1, wherein the weakly basic anion exchange resin constituting the mixed-bed ion exchange resin has the exchange capacity fraction of quaternary ammonium of 5 to 10%.

3. The method for purifying the allulose-containing solution of claim 1, wherein a mixed volume ratio of the strongly acidic cation exchange resin and the weakly basic anion exchange resin constituting the mixed-bed ion exchange resin is 1 : 1 to 1 : 4.

4. The method for purifying the allulose-containing solution of any one of claims 1 to 3, wherein the allulose-containing solution injected into the ion exchange resin column has a sugar solid concentration of 5 to 50 Brix, a pH of 3.5 to 5.5, an electrical conductivity of 5 to 80 *µs*/cm, and an allulose content of 90 to 99% (w/w) based on the total weight of a solid content.

5. A method for purifying an allulose-containing solution comprising obtaining an allulose-containing solution ion-purified by passing the allulose-containing solution through a first ion exchange resin column filled with a strongly acidic cation exchange resin, a second ion exchange resin column filled with a weakly basic anion exchange resin, and a third ion exchange resin column filled with a mixed-bed ion exchange resin, wherein
the mixed-bed ion exchange resin is a mixture of the strongly acidic cation exchange resin and the weakly basic anion exchange resin, and
the weakly basic anion exchange resin filled in the second ion exchange resin column and the weakly basic anion exchange resin constituting the mixed-bed ion exchange resin have exchange capacity fractions of quaternary ammonium of 0 to 10%.

6. The method for purifying the allulose-containing solution of claim 5, wherein the weakly basic anion exchange resin filled in the second ion exchange resin column has the exchange capacity fraction of quaternary ammonium of 0%.

7. The method for purifying the allulose-containing solution of claim 5, wherein the weakly basic anion exchange resin constituting the mixed-bed ion exchange resin has the exchange capacity fraction of quaternary ammonium of 5 to 10%.

8. The method for purifying the allulose-containing solution of claim 5, wherein a mixed volume ratio of the strongly acidic cation exchange resin and the weakly basic anion exchange resin constituting the mixed-bed ion exchange resin is 1 : 1 to 1 : 4.

9. The method for purifying the allulose-containing solution of claim 5, wherein a volume ratio of the strongly acidic cation exchange resin filled in the first ion exchange resin column, the weakly basic anion exchange resin filled in the second ion exchange resin column, and the mixed-bed ion exchange resin filled in the third ion exchange resin column is 1 : (0.5 to 2) : (0.5 to 2).

10. The method for purifying the allulose-containing solution of claim 5, wherein the allulose-containing solution injected into the first ion exchange resin column has a sugar solid concentration of 25 to 65 Brix, a pH of 3.5 to 5.5, an electrical conductivity of 20 to 400 *µs*/cm, and an allulose content of 10 to 50% (w/w) based on the total weight of a solid content.
